(19) Europäisches Patentamt
European Patent Office
Office européen des brevets

(11) EP 0 475 444 B1

(12) **EUROPÄISCHE PATENTSCHRIFT**

(45) Veröffentlichungstag und Bekanntmachung des Hinweises auf die Patenterteilung:
**14.07.1999 Patentblatt 1999/28**

(51) Int Cl.⁶: **C09K 19/34**, C09K 19/40, C09K 19/42, C07D 239/28, C07D 405/12, C07F 7/08

(21) Anmeldenummer: **91115584.4**

(22) Anmeldetag: **13.09.1991**

(54) **Die Verwendung von 4-Fluor-pyrimidin-Derivaten als Komponente für ferroelektrische Flüssigkristallmischungen und ihre Herstellung**

Use of 4-fluoro-pyrimidine derivatives as components for ferroelectric liquid crystal mixtures and process to prepare these compounds

Utilisation de derivés de fluoro-4-pyrimidines comme composants de mélanges ferroélectriques de cristaux liquides et procédé de synthèse de ces composés

(84) Benannte Vertragsstaaten:
**CH DE DK ES FR GB IT LI NL**

(30) Priorität: **14.09.1990 DE 4029165**
**27.09.1990 DE 4030582**

(43) Veröffentlichungstag der Anmeldung:
**18.03.1992 Patentblatt 1992/12**

(73) Patentinhaber: **HOECHST AKTIENGESELLSCHAFT**
**65926 Frankfurt am Main (DE)**

(72) Erfinder:
- **Wingen, Rainer, Dr.**
  **W-6234 Hattersheim am Main (DE)**
- **Illian, Gerhard, Dr.**
  **W-6000 Frankfurt am Main (DE)**

(74) Vertreter: **Bardehle, Heinz, Dipl.-Ing. et al**
**Patent- und Rechtsanwälte**
**Bardehle . Pagenberg . Dost . Altenburg . Geissler . Isenbruck**
**Theodor-Heuss-Anlage 12**
**68165 Mannheim (DE)**

(56) Entgegenhaltungen:
**EP-A- 0 154 840** **EP-A- 0 158 137**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 475 444 B1

## Beschreibung

Die ungewöhnliche Kombination von anisotropem und fluidem Verhalten der Flüssigkristalle hat zu ihrer Verwendung in einer Vielzahl verschiedener elektro-optischer Schalt- und Anzeigevorrichtungen geführt. Dabei können ihre elektrischen, magnetischen, elastischen und/oder thermischen Eigenschaften zu Orientierungsänderungen genutzt werden. Optische Effekte lassen sich beispielsweise mit Hilfe der Doppelbrechung, der Einlagerung dichroitisch absorbierender Farbstoffmoleküle ("guest-host mode") oder der Lichtstreuung erzielen.

Zur Erfüllung der ständig steigenden Praxisanforderungen auf den verschiedenen Anwendungsgebieten besteht laufend ein Bedarf an neuen verbesserten Flüssigkristall ("liquid crystal")-Mischungen und somit auch an einer Vielzahl mesogener Verbindungen unterschiedlicher Struktur. Dies gilt sowohl für die Gebiete, bei denen nematische LC-Phasen verwendet werden, als auch für solche mit smektischen LC-Phasen.

Auf besonderes Interesse sind in den letzten Jahren ferroelektrische flüssigkristalline Mischungen (FLC-Mischungen) gestoßen (siehe z.B. Lagerall et al. "Ferroelectric Liquid Crystals for Display", SID Symposium; October Meeting 1985, San Diego, Ca. USA).

Für die praktische Verwendung von ferroelektrischen Flüssigkristallen in elektrooptischen Anzeigen werden chirale, geneigt-smektische Phasen wie z.B. $S_c^*$ Phasen benötigt [siehe R.B. Meyer, L. Liebért, L, Strzelecki und P. Keller, J. Physique <u>36</u>, L-69 (1975)], die über einen großen Temperaturbereich stabil sind. Dieses Ziel kann man mit Verbindungen erreichen, die selbst solche Phasen, z.B. $S_c^*$ Phasen, ausbilden oder aber, indem man nicht chirale geneigt-smektische Phasen ausbildende Verbindungen mit optisch aktiven Verbindungen dotiert [siehe M. Brunet, Cl. Williams, Ann. Phys. <u>3</u>, 237 (1978)].

Da der flüssigkristalline Temperaturbereich der meisten ferroelektrischen Flüssigkristallsysteme zu klein ist, besteht laufend ein Bedarf an neuen Komponenten und neuen Flüssigkristallmischungen, die eine $S_c$-Phase nahe Raumtemperatur ausbilden.

In EP-B 0 158 137 werden 4-Fluorpyrimidine als Verbindungen und als Mischungskomponenten allgemein beschrieben. Sie weisen jedoch keine oder nur eine geringe Tendenz zur Ausbildung smektischer Phasen auf.

Die vorliegende Erfindung betrifft die Verwendung von speziellen 4-Fluorpyrimidin-Derivaten als Komponente für eine Flüssigkristallmischung, wobei man mindestens ein 4-Fluorpyrimidin der allgemeinen Formel (I) als Komponente in einer ferroelektrischen Flüssigkristallmischung bestehend aus 2 bis 20, vorzugsweise 2 bis 15, Komponenten einsetzt.

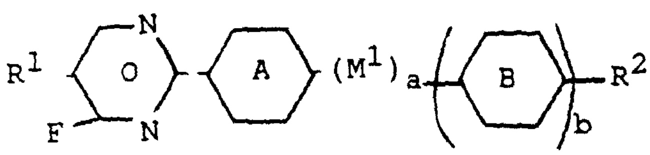 (I)

Die Symbole haben hierbei folgende Bedeutung:

$R^1$, $R^2$ unabhängig voneinander geradkettiges oder verzweigtes Alkyl mit 1 bis 16 C-Atomen, wobei ein oder zwei nicht benachbarte $-CH_2$-Gruppen durch -O-,-S-, -CO-, -CO-O-, -O-CO-, -CH=CH-, -OC(O)O-, -C=C-, 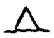 oder $-Si(CH_3)_2$- ersetzt sein können, oder eine der folgenden Gruppen:

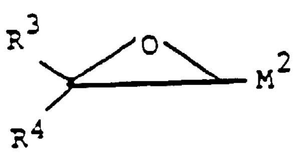 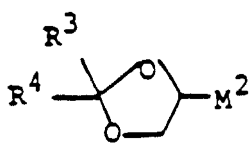 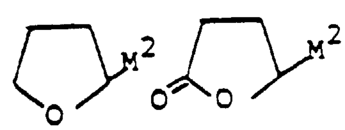

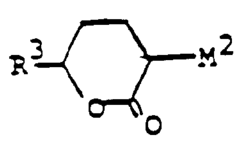 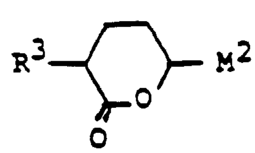 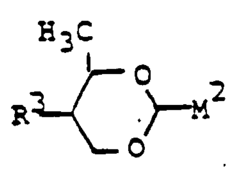

$R^3$, $R^4$ unabhängig voneinander H oder Alkyl mit 1 bis 16 oder Alkenyl mit 2 bis 16 C-Atomen, oder $R^3$ und $R^4$ zusammen $-(CH_2)_4$ oder $-(CH_2)_5$

$-M^1-$ -CO-O, -O-CO-, $-CH_2$-O-, -O-$CH_2$-, -C≡C-, -CH=CH-, $-CH_2$-$CH_2$-, $-CH_2CH_2CH_2CH_2$-, $-CH_2CH_2CH_2O$-

$-M^2-$ $-CH_2$-O-,

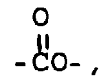

eine Einfachbindung, -O-$CH_2$-,

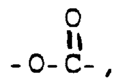

a, b unabhängig voneinander Null oder Eins, unter der Bedingung, daß a Null ist, wenn b Null ist,

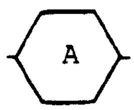 , 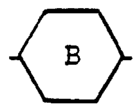

unabhängig voneinander 1,4-Phenylen, 1,4-Cyclohexylen, 2,5-Pyridin-diyl, (1,3,4)Thiadiazol-2,5-diyl

In einer bevorzugten Ausführungsform haben die Symbole in Formel (I) folgende Bedeutung:

$R^1$, $R^2$ unabhängig voneinander Alkyl mit 1 bis 16 C-Atomen, wobei eine $-CH_2$-Gruppe durch -O-, -CO-O-, -O-CO-, 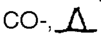 oder $Si(CH_3)_2$- ersetzt sein kann, oder

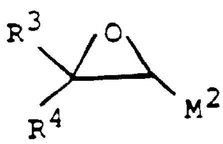

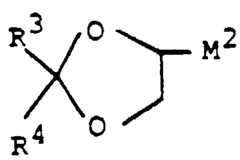

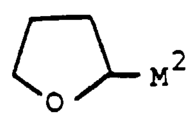

$-M^1-$ CO-O, O-CO, $CH_2O$ oder $OCH_2$

$-M^2-$ CO-O, $CH_2O$

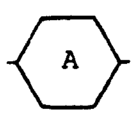 , 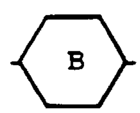

unabhängig voneinander 1,4-Phenylen oder 1,4-Cyclohexylen

$R^3$, $R^4$ beide H oder $C_1$-$C_5$-Alkyl oder $R^3$ und $R^4$ zusammen $-(CH_2)_5$-

a, b unabhängig voneinander Null oder Eins, unter der Bedingung, daß a Null ist, wenn b Null ist.

Besonders bevorzugt bedeuten $R^1$, $R^2$ unabhängig voneinander Alkyl mit 5 bis 12 C-Atomen, wobei eine $-CH_2$-Gruppe durch -O- und eine weitere $-CH_2$-Gruppe, insbesondere die endständige $CH_2$-Gruppe des Alkyls, durch 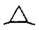 ersetzt sein kann, oder

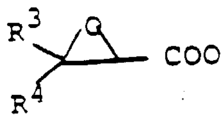

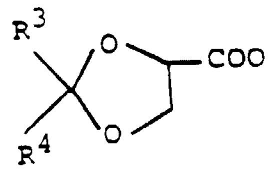

$-M^1-$ CO-O oder O-CO,

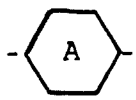 **1,4-Phenylen,**

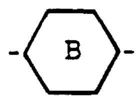 **1,4-Cyclohexylen und**

$R^3$, $R^4$ beide gleich H oder $C_1$-$C_5$-Alkyl oder $R^3$ und $R^4$ zusammen $-(CH_2)_5-$ und

a=b Null oder Eins.

Die Erfindung betrifft ferner eine ferroelektrische Flüssigkristallmischung bestehend aus 2 bis 20 Komponenten, die als eine Komponente mindestens ein 4-Fluor-pyrimidin der allgemeinen Formel (I) enthält.

Diese FLC-Mischung enthält im allgemeinen 0,1 bis 80, vorzugsweise 1 bis 60, insbesondere 1 bis 20 Mol-% der Verbindung der Formel (I). Sind mehrere der Verbindungen in der Mischung enthalten, so liegt der Gesamtgehalt bei 0,1 bis 80 Mol-%, vorzugsweise bei 1 bis 60 Mol-%.

Die anderen Bestandteile der Mischungen werden vorzugsweise ausgewählt aus den bekannten Verbindungen mit nematischen, cholesterischen und/oder geneigt/smektischen Phasen, dazu gehören beispielsweise Thiadiazol, Difluorphenyle, Fluorpyridine, Pyridine, Biphenyle, Terphenyle, Phenylcyclohexane, Cyclohexylbiphenyle, Pyrimidine, Zimtsäureester, Cholesterinester oder verschieden überbrückte Ester von p-Alkylbenzoesäuren.

Die beschriebenen Flüssigkristallmischungen lassen sich in elektrooptischen Schalt- und Anzeigevorrichtungen (FLC-Lichtventilen bzw. Displays) vorteilhaft einsetzen und führen z.B. zu einer Verkürzung der Schaltzeit und zu Verbreitungen der nematischen Phase. Diese weisen u.a. folgende Bestandteile auf: eine flüssigkristalline Mischung (enthaltend ein 4-Fluorpyrimidin), Trägerplatten (z.B. aus Glas oder Kunststoff), beschichtet mit transparenten Elektroden (zwei Elektroden), mindestens eine Orientierungsschicht, Abstandhalter, Kleberahmen, Polarisatoren, sowie für Farbdisplays dünne Farbfilterschichten. Weiter mögliche Komponenten sind Antireflex-, Passivierungs-, Ausgleichs- und Sperrschichten sowie elektrisch nichtlineare Elemente, wie z.B. Dünnschichttransistoren (TFT) und Metall-Isolator-Metall(MIM)-Elemente. Im Detail ist der allgemeine Aufbau von Flüssigkristalldisplays bereits in einschlägigen Monographien beschrieben (z.B. E. Kaneko, "Liquid Crystal Displays", KTK, Scientific Publishers, 1987, Seiten 12-30 und 163-172).

Die 4-Fluor-pyrimidine der Formel (I) sind chemisch, photochemisch und thermisch stabil; sie weisen vorzugsweise sowohl $S_c$-Phasen als auch nematische Phasen auf. Besonders bevorzugt sind dabei die 5-Alkyloxy-2-(4-alkyloxy)phenyl-4-fluor-pyrimidine, die neben der $S_c$-Phase auch noch niedrigere Schmelzpunkte als ihre nichzt fluorierten Analogen aufweisen. Vorzugsweise unterdrückt die Fluor-Substitution ferner die Bildung von höhergeordneten Phasen.

Die Erfindung betrifft ein Verfahren zur Herstellung von 4-Fluor-pyrimidin-Derivaten, insbesondere solcher der Formel (I), wobei man Pyrimidin-4-on-Verbindungen der Formel (II) mit einem Aminofluorosulfuran der Formel (III) zur Reaktion bringt,

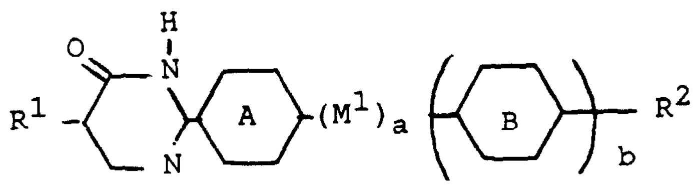

(II)

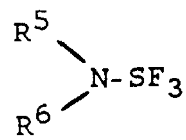

(III)

wobei

$R^5$ und $R^6$ unabhängig voneinander $C_1$- bis $C_{10}$-Alkyl, oder $R^5$ und $R^6$ zusammen $(-CH_2)_c(-O)_d(-CH_2)_e$ bedeuten, wobei

c = eine ganze Zahl von 1 bis 5,

d = Null oder 1 und

e = eine ganze Zahl von 1 bis 5 sind. Dabei wird eine

Pyrimidin-4-on-Verbindung (II) - die Herstellung erfolgt in Analogie zu Boller et al., Z. Naturf. 33 b (1978) - mit einem Fluorierungsreagenz direkt zu (I) umgesetzt. Gegenüber dem Verfahren der EP-B 0 158 137, Pyrimidin-4-on mit $POCl_3$ in die entsprechenden Chlorverbindungen zu überführen und diese dann mit einem fluorierenden Mittel zu den Fluorverbindungen umzusetzen, hat das erfindungsgemäße Verfahren den Vorteil, die Stufe der Chlorierung - die häufig mit Dunkelfärbungen und Bildung schwer abtrennbarer Verunreinigungen einhergeht - zu vermeiden. Prinzipiell ist das beschriebene Fluorierungsverfahren auf andere Pyrimidin-4-on-Verbindungen allgemein anwendbar.

Bevorzugt werden als Fluorierungsmittel Aminofluorosulfurane, bei denen in Formel (III) $R^5$ und $R^6$ $C_1$- bis $C_3$-Alkyl, oder $R^5$ und $R^6$ zusammen die Gruppe $-(CH_2)_2-O-(CH_2)_2-$ bedeuten, (siehe z.B. M. Hudlicky in Org. Reactions 53, 513). Besonders bevorzugt werden Diethylaminoschwefeltrifluorid und Morpholinoschwefeltrifluorid eingesetzt.

Die Erfindung wird durch die nachfolgenden Beispiele näher erläutert.

Beispiel 1

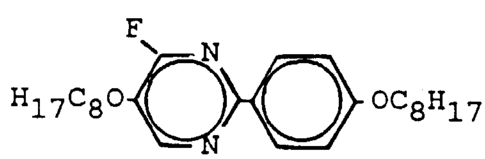

4-Fluor-5-octyloxy-2-(4-octyloxy-phenyl)pyrimidin

Darstellung:

Eine Suspension von 2.1 g 5-Octyloxy-2-(4-octyloxy-phenyl) pyrimidin-4-on (hergestellt z.B. nach Boller et al., Z. Naturf. 33b, 433 (1978)) in 30 ml Dichlormethan wird bei 0°C binnen 10 Minuten mit einer Lösung von 0.7 ml Diethylaminoschwefeltrifluorid (DAST) in 10 ml Dichlormethan versetzt. Nach 8 h bei 40°C wird mit wäßriger Natriumhydrogencarbonatlösung versetzt. Die organische Phase liefert nach Chromatographie und Umkristallisation aus Acetonitril 0.7 g farblose Kristalle. Phasenfolge X 43,5 $S_c$ 52 N 64,2 I

Vergleichsbeispiel 1b

Die unfluorierte Vergleichsverbindung weist mit 51°C einen Schmelzpunkt auf, der um 7°C höher liegt.

Analog werden erhalten:

Beispiel 2

4-Fluor-5-heptyl-2-(4-octyloxy-phenyl)pyrimidin
Phasenfolge X 40 N 43.8 I

Beispiel 3

4-Fluor-5-octyl-2-(4-octyloxy-phenyl)pyrimidin
Phasenfolge X 37 N 41,5 I

Beispiel 4

4-Fluor-5-nonyl-2-(4-octyloxy-phenyl)pyrimidin
Phasenfolge X 34 N 45 I

Beispiel 5

5-Decyl-4-fluor-2-(4-octyloxy-phenyl)pyrimidin
Phasenfolge X 36 N 43 I

Beispiel 6

2-(4-Ethoxy-phenyl)-4-fluor-5-octyloxy-pyrimidin
Phasenfolge X 70,5 (N) 55 I

Beispiel 7

5-[4-(Butyldimethylsilyl)butyloxy]-4-fluor-2-(4-octyloxyphenyl)pyrimidin
Phasenfolge: X 12.4 N 16 I

Beispiel 8

4-Fluor-5-octyloxy-2-[4-(4-trans-pentyl-cyclohexyl)phenyl]pyrimidin
Phasenfolge X 71 ($S_c$) 61 N 148 I

Vergleichsbeispiel 8b

Die unfluorierte Vergleichssubstanz weist bis 83°C eine höher geordnete smektische Phase auf. Offensichtlich führt die Fluorsubstitution zu einer Unterdrückung der höher geordneten smektischen Phase und damit zu einer Ausweitung der $S_c$-Phase nach tiefen Temperaturen.

Beispiel 9

2-[4-(4-Cyclopropylbutyloxy)phenyl]-4-fluor-5-octyloxypyrimidin
Phasenfolge X (45 N) 62 I

Beispiel 10

2-[4-(8-Cyclopropyloctyloxy)phenyl]-4-fluor-5-octyloxypyrimidin
Phasenfolge X 51 ($S_c$ 45 N 55) I

Beispiel 11

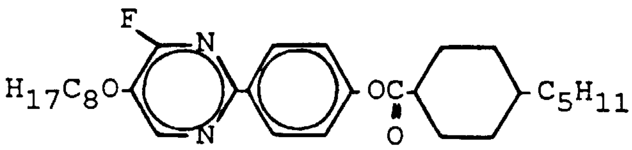

4-Fluor-5-octyloxy-2-[4-(4-trans-pentylcyclohexylcarbonyloxy)phenyl]pyrimidin

Darstellung:

Eine Lösung von 1.6 g 5-Octyloxy-2-[4-(4-transpentylcyclohexylcarbonyloxy)phenyl]pyrimidin-4-on (erhalten durch Umsetzung von 2-(4-Hydroxy-phenyl)-5-octyloxy-pyrimidin-4-on - Herstellung nach Boller et al. - mit trans-4-Pentylcyclohexancarbonsäure mittels Dicyclohexylcarbodiimid) in 40 ml Dichlormethan wird mit 0.45 ml Diethylaminoschwefeltrifluorid versetzt und 16 h bei 20°C gehalten. Nach Aufarbeitung wie in Beispiel 1 und Umkristallisation aus n-Hexan werden 0.3 g farblose Kristelle erhalten.
Phasenfolge X 80 $S_c$ 87 N 173 I
Diese $S_c$-Phase ist bis zur Kristallisation bei 60 existent.

Vergleichsbeispiel 11b

Die unfluorierte Vergleichssubstanz weist hingegen bei 74°C eine höher geordnete smektische Phase auf, die offensichtlich durch die Fluorierung unterdrückt wird.

Analog werden erhalten:

Beispiel 12

4-Fluor-5-octyl-2-[4-(4-trans-pentylcyclohexylcarbonyloxy) phenyl]pyrimidin
Phasenfolge X 78 N 137 I

Beispiel 13

(2R,3R)-3-Propyloxirancarbonsäure-[4-(4-fluor-5-octyloxypyrimidin-2-yl)phenyl]ester
Drehwert $[\alpha]_D^{20}$= -24.9° (c = 2, 1,2-Dichlorethan)
Phasenfolge X 90 I

Beispiel 14

2-(Octyloxyphenyl)-4-fluor-5-(4'-trans-pentyl-cyclohexyl-carbonyloxy)-pyrimidin
Phasenfolge: X 61 $S_c$ 77 N 188 I

Vergleichsbeispiel 14b

2-Dodecyloxyphenyl-5-(4'- trans-pentyl-cyclohexan-carbonyloxy)-pyrimidin
Phasenfolge: X 90 $S_A$ 185 N 188

Die erfindungsgemäße Substanz weist einen niedrigen Schmelzpunkt auf und anstelle der $S_A$-Phase beobachtet man eine $S_c$-Phase. Die Fluor-Substitution führt zu einer Unterdrückung der orthogonalen $S_A$-Phase und zu einer Verstärkung der geneigten $S_c$-Phase.

Beispiel 15

4-Fluor-2-(4-trans-pentylcyclohexyl)-pyrimidin-5-carbonsäureethylester

X 37 I

Beispiel 16

4-Fluor-2-(4-trans-pentylcyclohexyl)-pyrimidin-5-carbonsäure-(4-octyloxyphenyl)ester
X 80 $S_c$ 98 $S_A$ 118 N 143 I

Beispiel 17

4-Fluor-2-(4-trans-pentylcyclohexyl)-pyrimidin-5-carbonsäure[4-(5-octyl-pyrimidin-2-yl)phenyl]ester
X 120 N 233 I

Anwendungsbeispiel 1

Eine ferroelektrische Mischung besteht aus den folgenden Komponenten

1418 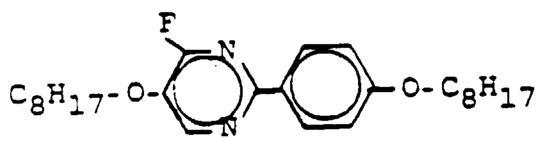 32.1 Mol-%

1465 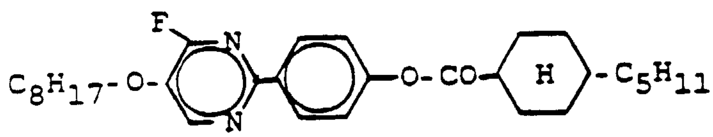 4.4 Mol.-%

1438 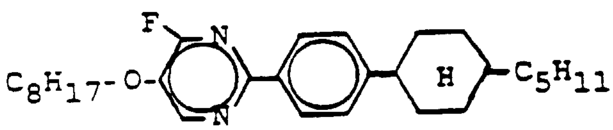 9.1 Mol.-%

546 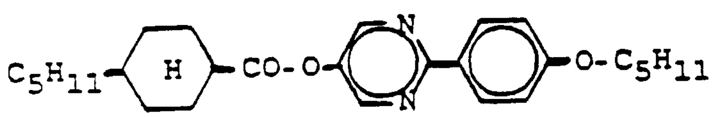 43.0 Mol.-%

106 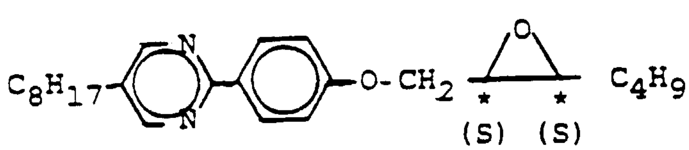 4.2 Mol.-%

459 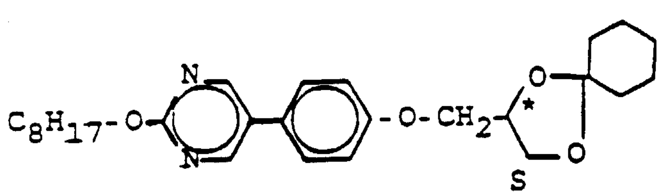 2.5 Mol.-%

723 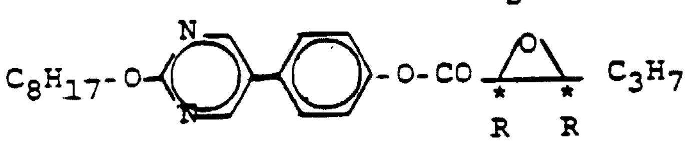 4.7 Mol.-%

und weist folgende Phasenfolge $S_c^*$ 84 $S_A^*$ 97 N* 125 I auf.

Diese Mischung schaltet bei einer Temperatur von 60°C bei einer Feldstärke von 10 V µm$^{-1}$ in einer 2 µm dicken Zelle mit einer Schaltzeit von 140 µsec.

Anwendungsbeispiel 2

Eine Mischung besteht aus den Komponenten

| | |
|---|---|
| 5-Octyloxy-2-(4-hexyloxy-phenyl)-pyrimidin | 8,6 Mol-% |
| 5-Octyloxy-2-(4-butyloxy-phenyl)-pyrimidin | 9,0 Mol.-% |
| 5-Octyloxy-2-(4-decyloxy-phenyl)-pyrimidin | 7,2 Mol.-% |
| 5-Octyloxy-2-(4-octyloxy-phenyl)-pyrimidin | 4,0 Mol.-% |
| 5-Octyl-2-(4-hexyloxy-phenyl)-pyrimidin | 13,7 Mol.-% |
| 5-Octyl-2-(4-octyloxy-phenyl)-pyrimidin | 12,3 Mol.-% |
| 5-Octyl-2-(4-decyloxy-phenyl)-pyrimidin | 9,2 Mol.-% |
| trans-4-Pentyl-cyclohexancarbonsäure-(4'-(5-dodecylpyrimidin-2-yl)-phenylester | 13,6 Mol.-% |
| 4-Fluor-5-octyloxy-2-(4-octyloxy-phenyl)-pyrimidin | 4,9 Mol.-% |
| | Mol.-% |
| (2S,3S)-2-(4-Octyl-pyrimidin-2yl-)-phenyloxy)-methyl-3-butyl-oxiran | 5,7 Mol.-% |
| (2R,3R)-3-propyl-oxiran-2-arbonsäure-(4-(2-Octyloxy-pyrimidin-5-yl)-phenyl)ester | 6,4 Mol.-% |
| (S)-4-(2-Octyloxypyrimidin-5-yl)-phenylspiro-(1,3-dioxolan-2,1-cyclohexan)4-yl)-methylether | 3,4 Mol.-% |
| 4,7,13,16,21,24,Hexaoxa-1,10-diazabicyclo-(8,8,8)hexacosan | 1,0 Mol.-% |
| 2,5,8,15,18,21-Hexaoxatricyclo(20,4,0,0)hexacosan | 1,0 Mol.-% |

zeigt folgende flüssigkristalline Phasenbereiche:

$S_c$ 58 $S_A$ 62N 80 I

und weist bei 25°C eine spontane Polarisation von 27,5 nC/cm auf.

Die ferroelektrische Mischung schaltet in einer 2 µm dicken mit Polyimid beschichteten Zelle bei einem angelegte Pulsfeldstärke von 10 $V\mu m^{-1}$ mit einer Pulsbreite von 114 µs.

Dieses Beispiel belegt, daß mit den erfindungsgemäßen Verbindungen schnell schaltende ferroelektrische Mischungen hergestellt werden können.

Anwendungsbeispiel 3

a) Eine Mischung besteht aus den Komponenten

| | |
|---|---|
| 5-Octyloxy-2-(4-hexyloxy-phenyl)-pyrimidin | 8,6 Mol-% |
| 5-Octyloxy-2-(4-butyloxy-phenyl)-pyrimidin | 9,0 Mol.-% |
| 5-Octyloxy-2-(4-decyloxy-phenyl)-pyrimidin | 7,2 Mol.-% |
| 5-Octyloxy-2-(4-octyloxy-phenyl)-pyrimidin | 4,0 Mol.-% |
| 5-Octyl-2-(4-hexyloxy-phenyl)-pyrimidin | 13,7 Mol.-% |
| 5-Octyl-2-(4-octyloxy-phenyl)-pyrimidin | 12,3 Mol.-% |
| 5-Octyl-2-(4-decyloxy-phenyl)-pyrimidin | 9,2 Mol.-% |
| trans-4-Pentyl-cyclohexancarbonsäure-(4'-(5-dodecylpyrimidin-2-yl)-phenylester | 13,6 Mol.-% |
| 4-Fluor-5-octyl-2-(4-octyloxy-phenyl)-pyrimidin | 4,9 Mol.-% |
| | Mol.-% |
| (2S,3S)-2-(4-Octyl-pyrimidin-2yl-)-phenyloxy)-methyl-3-butyl-oxiran | 5,7 Mol.-% |
| (2R,3R)-3-propyl-oxiran-2-arbonsäure-(4-(2-Octyloxy-pyrimidin-5-yl)-phenyl)ester | 6,4 Mol.-% |
| (S)-4-(2-Octyloxypyrimidin-5-yl)-phenylspiro-(1,3-dioxolan-2,1-cyclohexan)4-yl)-methylether | 3,4 Mol.-% |
| 4,7,13,16,21,24,Hexaoxa-1,10-diazabicyclo(8,8,8)hexacosan | 1,0 Mol.-% |
| 2,5,8,15,18,21-Hexaoxatricyclo(20,4,0,0)hexacosan | 1,0 Mol.-% |

zeigt folgende flüssigkristalline Phasenbereiche:

X -4 $S_c$ 57 $S_A$ 63 N 82 I

und weistbei25°C eine spontane Polarisation von 27 nC/cm auf. Die ferroelektrische Mischung schaltet in einer 2 µm dicken mit Polyimid beschichteten Zelle bei einem angelegte Pulsfeldstärke von 10 $V\mu m^{-1}$ mit einer Pulsbreite

von 93 µs.

b) Eine Vergleichsmischung, diesich von der obengenannten nur dadurch unterscheidet, daßsie keine Fluor-pyrimidin-Komponente enthält weist bei 25°C eine spontane Polarisation von 27 nC/cm auf und zeigt folgende flüssigkristalline Phasenbereiche:

X -4 $S_c$ 57 $S_A$ 68 N 83 I

Die ferroelektrische Mischung schaltet in einer 2 µm dicken mit Polyimid beschichteten Zelle bei einem angelegte Pulsfeldstärke von 10 $V\mu m^{-1}$ mit einer Pulsbreite von 115 µs. Diese Beispiel belegt, daß die erfindungsgemäßen Verbindungen in ferroelektrischen Mischungen zu einer Verkürzung der Schaltzeit beitragen.

## Patentansprüche

**1.** Verwendung eines Fluor-pyrimidin-Derivates als Komponente für Flüssigkristallmischungen, wobei man mindestens ein 4-Fluor-pyrimidin der allgemeinen Formel (I) als Komponente in einer ferroelektrischen Flüssigkristallmischung bestehend aus 2 bis 20 Komponenten einsetzt,

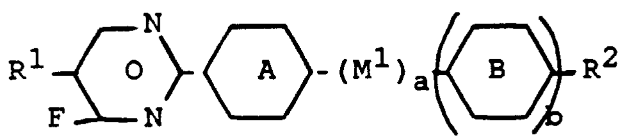 (I)

wobei die Symbole folgende Bedeutung tragen:

$R^1$, $R^2$ unabhängig voneinander geradkettiges oder verzweigtes Alkyl mit 1 bis 16 C-Atomen, wobei eine oder zwei nicht benachbarte $-CH_2$-Gruppen durch -O-,-S-, -CO-, -CO-O-, -O-CO-, -CH=CH-, -OC(O)O-, -C≡C-, 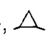 oder $-Si(CH_3)_2-$ ersetzt sein können, oder eine der folgenden Gruppen:

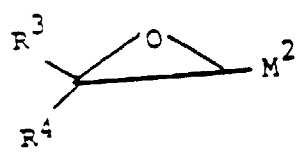
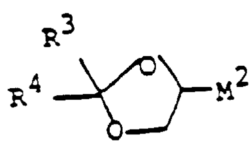
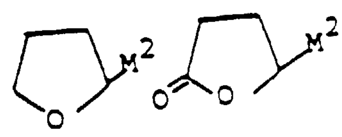

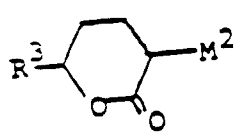
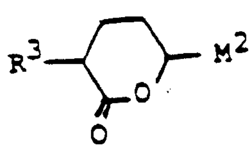
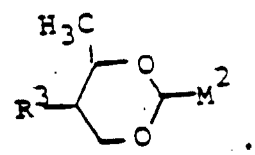

$R^3$, $R^4$ unabhängig voneinander H oder Alkyl mit 1 bis 16 oder Alkenyl mit 2 bis 16 C-Atomen, oder $R^3$ und $R^4$ zusammen $-(CH_2-)_4-$ oder $-(CH_2)_5-$

$-M^1-$ -CO-O, -O-CO-, $-CH_2-O-$, $-O-CH_2-$, -C≡C-, -CH=CH-, $-CH_2-CH_2-$, $-CH_2CH_2CH_2CH_2-$, $-CH_2CH_2CH_2O-$

$-M^2-$ $-CH_2-O-$, $-O-CH_2$,

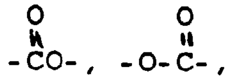

eine Einfachbindung

a, b unabhängig voneinander Null oder Eins, unter der Bedingung, daß a Null ist, wenn b Null ist,

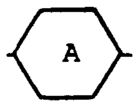 , 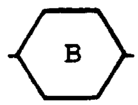

unabhängig voneinander 1,4-Phenylen, 1,4-Cyclohexylen, 2,5-Pyridin-diyl, (1,3,4)Thiadiazol-2,5-diyl

**2.** Verwendung gemäß Anspruch 1, dadurch gekennzeichnet, daß in Formel (I) die Symbole folgende Bedeutung haben:

$R^1$, $R^2$ unabhängig voneinander Alkyl mit 1 bis 16 C-Atomen, wobei die $-CH_2$-Gruppe durch -O-, -CO-O-, -O-CO-, 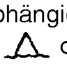 oder $-Si(CH_3)_2$- ersetzt sein kann, oder

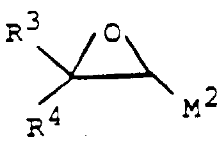

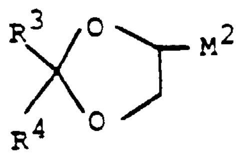

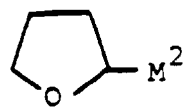

$-M^1-$ -CO-O, O-CO-, $CH_2O$ oder $-OCH_2-$

$-M^2-$ -CO-O-, oder $-CH_2O-$

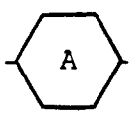 , 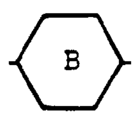

unabhängig voneinander 1,4-Phenylen oder 1,4-Cyclohexylen

$R^3$, $R^4$ beide H oder $C_1$-$C_5$-Alkyl oder $R^3$ und $R^4$ zusammen $-(CH_2)_5-$

a, b unabhängig voneinander Null oder Eins, unter der Bedingung, daß a Null ist, wenn b Null ist.

**3.** Verwendung gemäß Anspruch 1, dadurch gekennzeichnet, daß in Formel (I) die Symbole folgende Bedeutung haben:

$R^1$, $R^2$ unabhängig voneinander Alkyl mit 5 bis 12 C-Atomen, wobei eine $-CH_2$-Gruppe durch -O- und eine weitere Gruppe durch 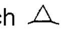 ersetzt sein kann, oder

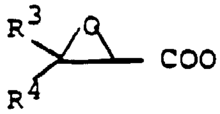

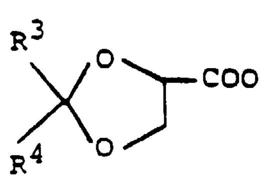

-$M^1$- -CO-O- oder -O-CO-,

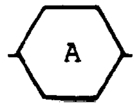 1,4-Phenylen,

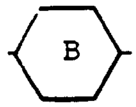 1,4-Cyclohexylen und

$R^3$, $R^4$ beide gleich H oder $C_1$-$C_5$-Alkyl oder $R^3$ und $R^4$ zusammen $-(CH_2)_5-$ und

a=b Null oder Eins.

**4.** Verwendung gemäß Anspruch 1, dadurch gekennzeichnet, daß in Formel (I) die Gruppen $R^1$ und $R^2$ unabhängig voneinander Alkyl mit 5 bis 12 C-Atomen bedeuten, wobei eine $-CH_2$-Gruppe durch -CO-O-, -O-CO- oder -O- ersetzt sein kann und wobei die terminale $-CH_2$-Gruppe durch 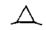 ersetzt ist.

**5.** Ferroelektrische Flüssigkristallmischung bestehend aus 2 bis 20 Komponenten, dadurch gekennzeichnet, daß sie als eine Komponente mindestens ein 4-Fluor-pyrimidin der allgemeinen Formel (I) aus Anspruch 1 enthält.

**6.** Ferroelektrisches Flüssigkristall-Schalt- und -Anzeigeelement enthaltend Trägerplatten, Elektroden, mindestens eine Orientierungsschicht, ein ferroelektrisches flüssigkristallines Medium sowie gegebenenfalls weitere Komponenten, dadurch gekennzeichnet, daß es als flüssigkristallines Medium eine ferroelektrische Flüssigkristallmischung gemäß Anspruch 5 enthält.

**7.** Verfahren zur Herstellung von 4-Fluor-pyrimidin-Derivaten der Formel (I) gemäß Anspruch 1, dadurch gekennzeichnet, daß man Pyrimidin-4-on-Verbindungen der Formel (II) mit einem Aminosulfuran der Formel (III) zur Reaktion bringt,

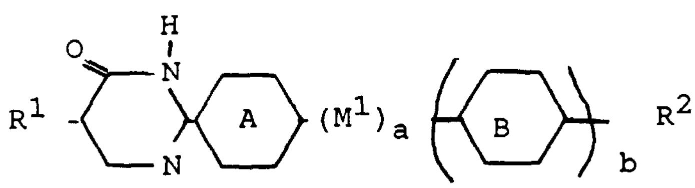 (II)

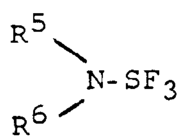 (III)

wobei
$R^5$ und $R^6$ unabhängig voneinander $C_1$- bis $C_{10}$-Alkyl, oder $R^5$ und $R^6$ zusammen $(-CH_2)_c(-O)_d(-CH_2)_e$ bedeuten, wobei
c = eine ganze Zahl von 1 bis 5,
d = Null oder 1 und
e = eine ganze Zahl von 1 bis 5 sind
und die Substituenten $R^1$, $M^1$, $R^2$, a, b,

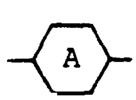 und 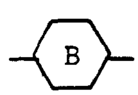

die in Anspruch 1 genannten Bedeutungen haben.

8. Verfahren gemäß Anspruch 7, dadurch gekennzeichnet, daß in Formel (III) $R^5$ und $R^6$ $C_1$- bis $C_3$-Alkyl, oder $R^5$ und $R^6$ zusammen die Gruppe $-(CH_2)_2-O-(CH_2)_2-$ bedeuten.

## Claims

1. Use of a fluoropyrimidine derivative as component for liquid crystal mixtures, at least one 4-fluoropyrimidine of the general formula (I) being employed as component in a ferroelectric liquid crystal mixture comprising 2 to 20 components,

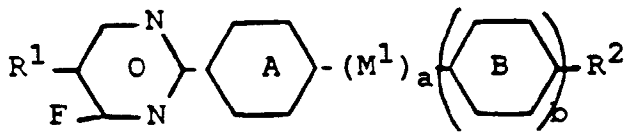

(I)

in which the symbols have the following meaning:

$R^1$ and $R^2$ independently of one another are straight-chain or branched alkyl having 1 to 16 carbon atoms, it being possible for one or two non-adjacent $-CH_2-$ groups to be replaced by -O-, -S-, -CO-, -CO-O-, -O-CO-, -CH=CH-, -OC(O)O-, -C≡C-, △ or $-Si(CH_3)_2-$, or are one of the following groups:

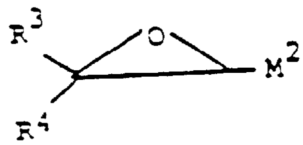

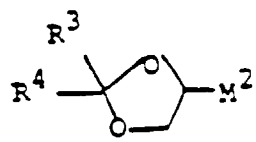

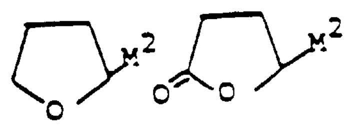

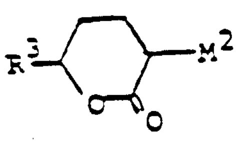

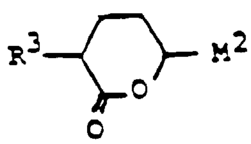

or

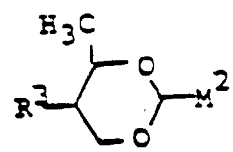

.

$R^3$ and $R^4$ independently of one another are H or alkyl having 1 to 16 carbon atoms or alkenyl having 2 to 16 carbon atoms, or $R^3$ and $R^4$ together are $-(CH_2)_4-$ or $-(CH_2)_5-$,

$-M^1-$ is -CO-O, -O-CO-, $-CH_2-O-$, $-O-CH_2-$, -C≡C-, -CH=CH-, $-CH_2-CH_2-$, $-CH_2CH_2CH_2CH_2-$, $-CH_2CH_2CH_2O-$

$-M^2-$ is $-CH_2-O-$, $-O-CH_2$,

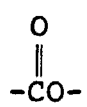

,

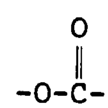

,

or a single bond

a and b independently of one another are zero or one, on condition that a is zero if b is zero, and

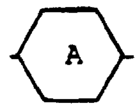 and 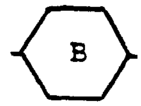

independently of one another are 1,4-phenylene, 1,4-cyclohexylene, 2,5-pyridinediyl or (1,3,4)thiadiazol-2,5-diyl.

**2.** The use as claimed in claim 1, wherein the symbols in formula (I) have the following meaning:

$R^1$ and $R^2$ independently of one another are alkyl having 1 to 16 carbon atoms, it being possible for the $-CH_2-$ group to be replaced by -O-, -CO-O-, -O-CO-, 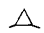 or $-Si(CH_3)_2-$, or are

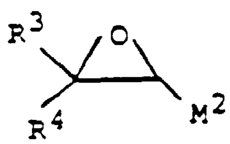 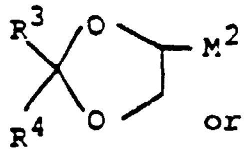 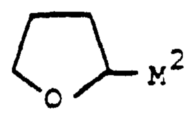

$-M^1-$ is -CO-O, O-CO-, $CH_2O$ or $-OCH_2-$

$-M^2-$ is -CO-O- or $-CH_2O-$

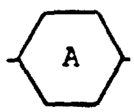 and 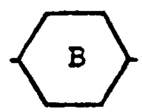

independently of one another are 1,4-phenylene or 1,4-cyclohexylene,

$R^3$ and $R^4$ are both H or $C_1$-$C_5$-alkyl, or $R^3$ and $R^4$ together are $-(CH_2)_5-$, and

a, b independently of one another are zero or one, on condition that a is zero if b is zero.

**3.** The use as claimed in claim 1, wherein the symbols in formula (I) have the following meaning:

$R^1$ and $R^2$ independently of one another are alkyl having 5 to 12 carbon atoms, it being possible for one $-CH_2-$ group to be replaced by -O- and for a further $-CH_2-$ group to be replaced by 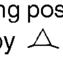, or are

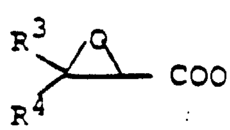 or 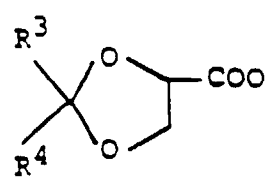

$-M^1-$ is -CO-O- or -O-CO-,

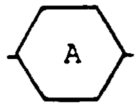 is 1,4-phenylene,

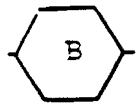 is 1,4-cyclohexylene and

$R^3$ and $R^4$ are both H or $C_1$-$C_5$-alkyl, or $R^3$ and $R^4$ together are $-(CH_2)_5-$, and

a=b and is zero or one.

**4.** The use as claimed in claim 1, wherein, in formula (I), the groups $R^1$ and $R^2$ independently of one another are alkyl having 5 to 12 carbon atoms, it being possible for one $-CH_2-$ group to be replaced by -CO-O-, -O-CO- or -O- and the terminal $-CH_2-$ group being replaced by 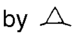

**5.** A ferroelectric liquid crystal mixture comprising 2 to 20 components, which mixture contains at least one 4-fluoropyrimidine of the general formula (I) from claim 1 as one component.

**6.** A ferroelectric liquid crystal switching and display element containing support plates, electrodes, at least one orientation layer, a ferroelectric liquid crystal medium and also, optionally, further components, which element contains a ferroelectric liquid crystal mixture as claimed in claim 5 as liquid crystal medium.

**7.** A process for the preparation of a 4-fluoropyrimidine derivative of the formula (I) as claimed in claim 1, which comprises reacting a pyrimidin-4-one compound of the formula (II) with an aminosulfurane of the formula (III)

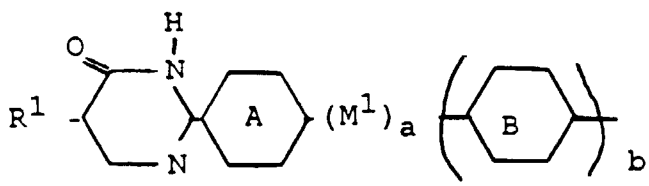

(II)

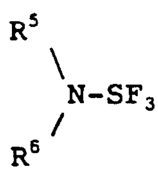

(III)

in which
$R^5$ and $R^6$ independently of one another are $C_1$-to $C_{10}$-alkyl, or $R^5$ and $R^6$ together are $(-CH_2)_c(-O)_d(-CH_2)_e$, in which
c = an integer from 1 to 5,
d = zero or 1 and
e = an integer from 1 to 5,
and the substituents $R^1$, $M^1$, $R^2$, a, b,

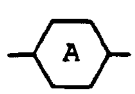 and 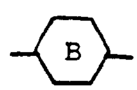

have the meanings indicated in claim 1.

**8.** The process as claimed in claim 7, wherein, in formula (III) $R^5$ and $R^6$ are $C_1$- to $C_3$-alkyl, or $R^5$ and $R^6$ together are the $-(CH_2)_2-O-(CH_2)_2-$ group.

## Revendications

**1.** Utilisation d'un dérivé fluoro-pyrimidine comme composant pour des compositions de cristaux liquides, dans laquelle on utilise au moins une 4-fluoro-pyrimidine de formule générale (I) comme composant dans une composition ferroélectrique de cristaux liquides constituée de 2 à 20 composants,

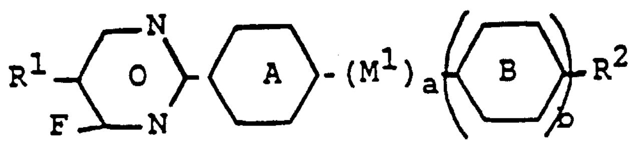

(I)

formule dans laquelle les symboles ont la signification suivante :

$R^1$, $R^2$ représentent indépendamment l'un de l'autre un groupe alkyle à chaîne linéaire ou ramifiée ayant 1 à 16 atomes de carbone, dans lequel un ou deux groupes $-CH_2-$ non voisins peuvent être remplacés par -O-, -S-, -CO-, -CO-O-, -O-CO-, -CH=CH-, -OC(O)O-, -C≡C-, △ ou $-Si(CH_3)_2-$, ou un des groupes suivants :

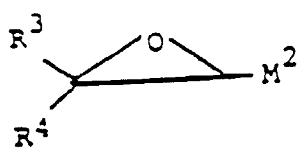
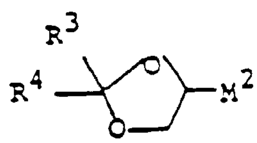
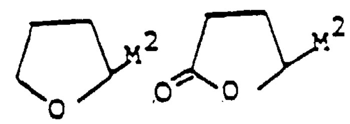

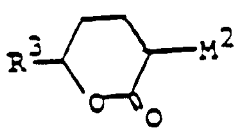
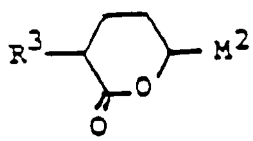
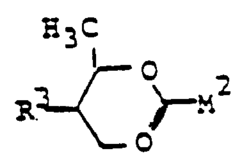

$R^3$, $R^4$ représentent indépendamment l'un de l'autre H ou un groupe alkyle ayant 1 à 16 atomes de carbone ou alcényle avec 2 à 16 atomes de carbone, ou $R^3$ et $R^4$ représentent ensemble $-(CH_2)_4-$ ou $-(CH_2)_5-$

$-M^1-$ représente -CO-O-, -O-CO-, $-CH_2-O-$, $-O-CH_2-$, -C≡C-, -CH=CH-, $-CH_2-CH_2-$, $-CH_2CH_2CH_2CH_2-$, $-CH_2CH_2CH_2O-$

$-M^2-$ représente $-CH_2-O-$, $-O-CH_2-$,

$$-\overset{\overset{O}{\|}}{C}O-,\quad -O-\overset{\overset{O}{\|}}{C}O-,$$

une simple liaison

a, b représentent indépendamment l'un de l'autre zéro ou un, à la condition que a soit nul si b est nul,

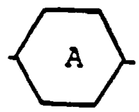 , 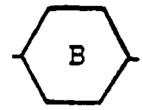

représentent indépendamment l'un de l'autre un groupe 1,4-phénylène, 1,4-cyclohexylène, 2,5-pyridin-diyle, (1,3,4)thiadiazol-2,5-diyle

**2.** Utilisation selon la revendication 1, caractérisée en ce que dans la formule (I) les symboles ont la signification suivante :

$R^1$, $R^2$ représentent indépendamment l'un de l'autre un groupe alkyle ayant 1 à 16 atomes de carbone, dans lequel les groupes $-CH_2-$ peuvent être remplacés par -O-, -CO-O-, -O-CO-, △ ou $-Si(CH_3)_2-$, ou

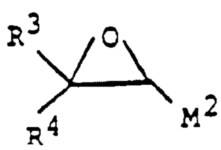 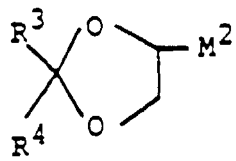 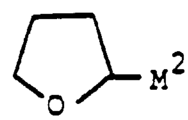

$-M^1-$ représente -CO-O-, -O-CO-, $-CH_2-O-$ ou $-O-CH_2-$
$-M^2-$ représente -CO-O- ou $-CH_2-O$

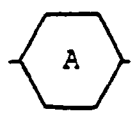 , 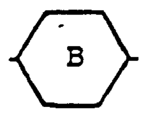

représentent indépendamment l'un de l'autre un groupe 1,4-phénylène ou 1,4-cyclohexylène

$R^3$, $R^4$ représentent tous les deux H ou un groupe alkyle en $C_1$-$C_5$, ou $R^3$ et $R^4$ représentent ensemble $-(CH_2)_5-$
a, b représentent indépendamment l'un de l'autre zéro ou un, à la condition que a soit nul si b est nul.

**3.** Utilisation selon la revendication 1, caractérisée en ce que dans la formule (I) les symboles ont la signification suivante :

$R^1$, $R^2$ représentent indépendamment l'un de l'autre un groupé alkyle ayant 5 à 12 atomes de carbone, dans lequel un groupe $-CH_2-$ peut être remplacé par -O-, et un autre groupe par △, ou

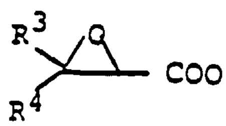 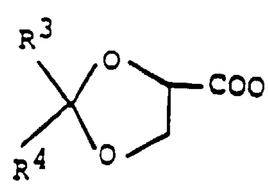

$-M^1-$ représente -CO-O- ou -O-CO-,

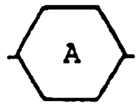 le 1,4-phénylène,

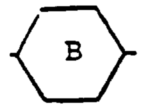 le 1,4-cyclohexylène et

$R^3$, $R^4$ représentent tous les deux H ou un groupe alkyle en $C_1$-$C_5$, ou $R^3$ et $R^4$ représentent ensemble $-(CH_2)_5$-
a = b représente zéro ou un.

**4.** Utilisation selon la revendication 1, caractérisée en ce que dans la formule (I) les groupes $R^1$ et $R^2$ représentent indépendamment l'un de l'autre un groupe alkyle ayant 5 à 12 atomes de carbone, dans lequel un groupe $-CH_2$- peut être remplacé par -CO-O-, -O-CO-, ou -O- et dans lequel le groupe $-CH_2$- terminal est remplacé par 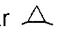 .

**5.** Composition de cristaux liquides ferroélectrique constituée de 2 à 20 composants, caractérisée en ce qu'elle contient comme composant au moins une 4-fluoro-pyridine de formule générale (I) selon la revendication 1.

**6.** Elément de circuit et d'affichage à cristaux liquides ferroélectrique contenant des plaques support, des électrodes, au moins une couche d'orientation, un milieu à cristal liquide ferroélectrique et éventuellement d'autres composants, caractérisé en ce qu'il contient comme milieu ferroélectrique une composition de cristaux liquides selon la revendication 5.

**7.** Procédé de préparation de dérivés 4-fluoro-pyrimidine de formule (I) selon la revendication 1, caractérisé en ce qu'on met à réagir des dérivés pyrimidin-4-one de formule (II) avec un aminosulfurane de formule (III),

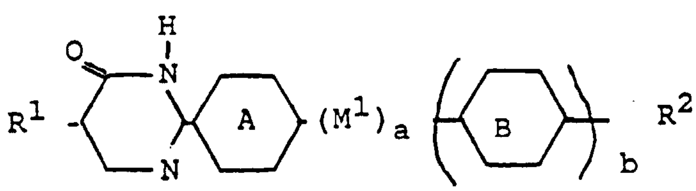 (II)

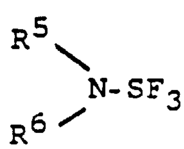 (III)

dans lesquelles
$R^5$ et $R^6$ représentent indépendamment l'un de l'autre un groupe alkyle en $C_1$ à $C_{10}$, ou $R^5$ et $R^6$ représentent ensemble $(-CH_2)_c(-O)_d(-CH_2)_e$; dans lesquelles
c = un nombre entier de 1 à 5,
d = zéro ou 1 et
e = un nombre entier de 1 à 5
et les substituants $R^1$, $M^1$, $R^2$, a, b,

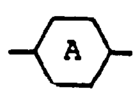 et 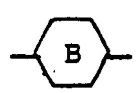

ont les significations citées à la revendication 1.

**8.** Procédé selon la revendication 7, caractérisé en ce que dans la formule (III) $R^5$ et $R^6$ représentent un groupe alkyle en $C_1$ à $C_3$, ou $R^5$ et $R^6$ représentent ensemble le groupe-$(CH_2)_2$-O-$(CH_2)_2$.